# EUROPEAN PATENT APPLICATION

(11) **EP 1 980 262 A1**
(43) Date of publication of application: **15.10.2008**
(21) Application number: 07702055.0
(22) Date of filing: 12.01.2007
(51) Int. Cl.: A61K 36/77

(54) **AN EXTRACT OF XANTHOCERAS SORBIFOLIA BUNGE AND EXTRACTION AND USES THEREOF**

(30) Priority: 12.01.2006 CN 200610009619
(71) Applicant: Liu, Tony, Heilongjiang 150086 (CN)
(72) Inventor: Liu, Tony, Heilongjiang 150086 (CN)
(74) Representative: De Gregori, Antonella
(86) International application number: PCT/CN2007/000123
(87) International publication number: WO 2007/079695

(57) **Abstract**

The present invention discloses a kind of Shinyleaf yellowhom extract mainly consisting of Shinyleaf yellowhom total saponins, which accounts for more than 5 0% of the total weight of the dried Shinyleaf yellowhom extract. The present invention also provides a method for preparing the above extract, as well as its use in the treatment of the urinary incontinence and frequent micturition due to senile dementia or urinary bladder degradation, children enuresis, pediatric mental retardation, brain aging, middle-aged and senile dementia and Parkinson's syndrome. The pharmaceutical preparations of the present invention include injections, tablets, capsules, soft capsules, oral liquid, drop pills, etc.

## Description

### Field of the Invention

The present invention relates to Shinyleaf yellowhorn extract, methods for extraction, and uses thereof in preparation of drug for treatment of urinary incontinence and frequent micturition due to senile dementia or urinary bladder degradation, and children enuresis.

### Background of the Invention

Shinyleaf yellowhorn, also known as Wenguanguo and Yanmugua, is the fruit of the perennial herb Xanthoceras sorbifolia Bunge (family: Soapberry; genus: Xanthoceras). The wild Xanthoceras sorbifolia's provenance is the northern part of China, currently Xanthoceras sorbifolia is mostly cultivated. Shinyleaf yellowhorn has been used mainly for the treatment of rheumarthritis, rheumatic calor internus and dermal rheumatism, and was recorded in the Chinese Pharmacopoeia in 1977.

In recent years, Shinyleaf yellowhorn and its alcohol extract has been proven to improve the learning and memory functions, and has been used for treatment of mental retardation and senile dementia. Journal of Medicine & Pharmacy of Chinese Minorities, 1997, vol.3(1) reported a clinical observation of directly using dried Shinyleaf yellowhorn for treating 120 cases of enuresis. CN1092991A reported the use of alcohol extract of Shinyleaf yellowhorn for improving brain function, and preparation thereof. However, it is difficult to extract the saponins from Shinyleaf yellowhorn. The content of total saponins in Shinyleaf yellowhorn extract obtained by existing techniques is generally 18 to 28%, and that from Shinyleaf yellowhom nutshells is 15 to 25% (see, CN1350001 'Nutshells for extraction of Shinyleaf yellowhorn saponin, crude fat, crude protein and sugar'). The existing method for evaluating the content of total saponins in Shinyleaf yellowhorn extract is poor in accuracy, which directly impeded dosage accuracy, quality control and therapeutic effect of the drugs.

Because the chemical components of Shinyleaf yellowhom are fairly complex, it is of great difficult to use the chromatographic technique, which is suitable for and widely adopted in industrial production, to obtain the high-content (>50%) total saponins from Shinyleaf yellowhom. Until now, there is yet no report about the preparation of S hinyleaf yellowhorn extract containing high-content (>50%) total saponins, nor method for the preparing. Shinyleaf yellowhom extract containing high-content total saponins by chromatographic technique.

Currently, there is no safe, effective and eutherapeutic drug for the treatment of urinary incontinence and frequent micturition due to senile dementia or urinary bladder degradation, children enuresis and pediatric mental retardation, brain aging, and middle-age and senile dementia. The present invention has addressed the need by providing Shinyleaf yellowhorn extract as a plant pharmaceutical preparation that has good therapeutic effects with no toxic and side effects.

### Description of the Invention

The purpose of the present invention is to provide a kind of Shinyleaf yellowhom extract with high content of total saponins. A second purpose of the present invention is to provide a method for the preparing said extract. Another purpose is to provide a method for measuring the content of total saponins in Shinyleaf yellowhom extract. Still another purpose is to deal with the use of Shinyleaf yellowhom extract in the preparation of drugs for the treatment of the urinary incontinence and frequent micturition due to senile dementia or urinary bladder degradation, and children enuresis. Yet another purpose is to provide a method for treatment of the above diseases with the above extract.

The above purposes are achieved by the following preferred embodiment:

The present invention provides a kind of Shinyleaf yellowhorn extract, comprising mainly the total saponins, which accounts for more than 50% of the gross weight of the dried Shinyleaf yellowhom extract (more than 60% in preferred embodiment, more preferably more than 70%, most preferably more than 80%, and even up to 90% or more). Saponin B in the Shinyleaf yellowhom extract accounts for more than 15%, more than 18% in preferred embodiment, more preferably more than 21 %, based on the total weight of dried Shinyleaf yellowhom extract.

The present invention also provides a method for preparing the above extract. The present inventor found that, it is possible to obtain Shinyleaf yellowhom extract containing more than 50% of total saponins by a unique extracting and refining technique.

The method for preparing Shinyleaf yellowhom extract comprises the following steps:
A. Raw materials of Shinyleaf yellowhom is dried and deoiled (preferably using an oil press) to get cake powder of Shinyleaf yellowhom. The cake powder is extracted with Extractant A or extracted by supercritical CO₂ extraction. Then the resultant extract is dried to get Shinyleaf yellowhom extract cream. Extractant A is selected from n-hexane, petroleum ether, diethyl ether and chloroform, with n-hexane prefered;
B. The Shinyleaf yellowhorn extract cream is extracted with Extractant B. The resultant liquid extracts are filtered, combined, added with water to dissolve, and then filtered to get Liquid Extract I. Extractant B may be methanol or ethanol;
C. Liquid Extract I is further extracted with Extractant C. The resultant liquid extracts are filtered, combined, added with water to dissolve, and then filtered to get Liquid Extract II. Extractant C may be selected from n-butanol, n-propanol, isopropanol, isobutanol, and pentanol, preferably n-butanol, n-propanol, and isopropanol;
D. Liquid Extract II is loaded onto the styrene macroporous resin column for adsorption process. Then eluted with methanol or ethanol solution. The eluate is collected, concentrated and dried to obtain refined Shinyleaf yellowhorn extract.

According to a preferred embodiment of the present invention, the method for preparing Shinyleaf yellowhorn extract comprises the following steps:
A. Raw materials of Shinyleaf yellowhom is dried and deoiled (preferably using an oil press) to get cake powder of Shinyleaf yellowhom. The cake powder is extracted with Extractant A or by supercritical CO₂ extraction. The resultant extract is dried to obtain Shinyleaf yellowhorn extract cream.
   In the case of extracted with Extractant A, it is prefered to extract with Extractant A at a weight/weight ratio of 1~5:1, preferably 2~3:1, and to perform the extraction procedure 2~6 times, preferably 3~5 times. The solvent is then recovered from the resultant extract under reduced pressure, and dried to obtain Shinyleaf yellowhom extract cream;
   In the case of extracted by supercritical CO₂ extraction, the cake powder is added to supercritical CO₂ to perform extraction. The technical parameters for supercritical CO₂ extraction are as follows: extraction pressure: 10~50MPa (preferably 20~30MPa); extraction temperature: 20~60°C (preferably 40~50°C); performed times: 2~5; entraining agent: ethanol, volume fraction: 60~95%, amount: 1~6% (preferably 2~4%).
B. The Shinyleaf yellowhorn extract cream is extracted with Extractant B for 1 or 2 times, each lasting about 2~4 hours, preferably with Extractant B of a concentration of 50~90% (preferably 55~85%, more preferably 65~80%) at a volume/weight ratio of 2~9:1 (preferably 4~8:1). The resultant liquid extracts are filtered and combined, and then alcohol is recovered under reduced pressure, preferably at 60~70°C, until there is no odor of alcohols. Added with 0.5~3 volumes (preferably 1~2 volumes) of water and filtered again to obtain Liquid Extract I.
C. The Liquid Extract I is added with Extractant C to perform extraction 1-3 times. It is preferred to use Extractant C of a volume/volume ratio of 0.5~3:1 (preferably 1~2:1), each for 2~4 hours. The resultant liquid extracts are filtered, combined, and then recover alcohol under reduced pressure (preferably at 60~70°C), until there is no odor of alcohols. Added with 0.2∼2 volumes (preferably 0.5~1 volumes) of water and filtered again to obtain Liquid Extract II.
D. Liquid Extract II is loaded onto the styrene macroporous resin column for adsorption process. It is preferred for adsorption process to perform for 10~60 min (more preferably for 20~40min). Eluted with 600~1500ml of water, prefered a flow rate of 1.0~4.0ml/min. Then eluted with 800~3000ml of 30~80% (preferably 40∼70 %, more preferably 60~70%) methanol or ethanol solution, preferably a flow rate of 0.5~2.0ml/min. A 20~30% of alcohol eluate is collected and recover alcohol under reduced pressure (preferably at 60~70°C), until there is no odor of alcohols. The resultant solution is concentrated and dried to obtain a refined Shinyleaf yellowhom extract.

The above-mentioned prefered extraction procedures is preferably thermal reflux extraction or ultrasonic extraction. The weight/volume ratio as used herein is kg/L.

According to an embodiment of the present invention, the styrene macroporous resin column as used herein can be polar, weak polar, or non-polar ones. Before the absorption process, the resin column can be pretreated with methanol or ethanol and washed with water before added with extractant. In particular embodiment of the present invention, the resin used can be any of the following: DA201 (polar macroporous polystyrene high-molecular polymers with the milky or light yellow opaque spherical particles, produced by the Tianjin University Chemical Plant), AB8 (non-polar macroporous polystyrene high-molecular polymers with the milky or light yellow opaque spherical particles, produced by the Tianjin AAA Plant), D101 (weak polar macroporous polystyrene high-molecular polymers with the milky or light yellow opaque spherical particles, produced by the Tianjin University Chemical Plant), HP20 (weak polar macroporous polystyrene high-molecular polymers with the milky opaque spherical particles, produced by the Japanese Mitsubishi Chemical Industrial Corporation), SP825 (weak polar macroporous polystyrene high-molecular polymers with the brown opaque spherical particles, produced by the Japanese Mitsubishi Chemical Industrial Corporation).

The experiments have shown that the total saponins of Shinyleaf yellowhom mainly consist of saponins A, B, C and D, and these components have a relatively stable ratio. It has been reported [see 'Progress in the study of chemical components of Shinyleaf yellowhom and their pharmacological actions', Journal of Shenyang Pharmaceutical University, 2 004, 21(6)] that the nucleus of Shinyleaf yellowhom total saponins is a class of compounds with oleanane-type pentacyclic triterpenoids. The molecular formula of the saponin B in Shinyleaf yellowhom is C₆₀H₉₂O₂₄ with a molecular weight of 1196, and its chemical structure is as follows:

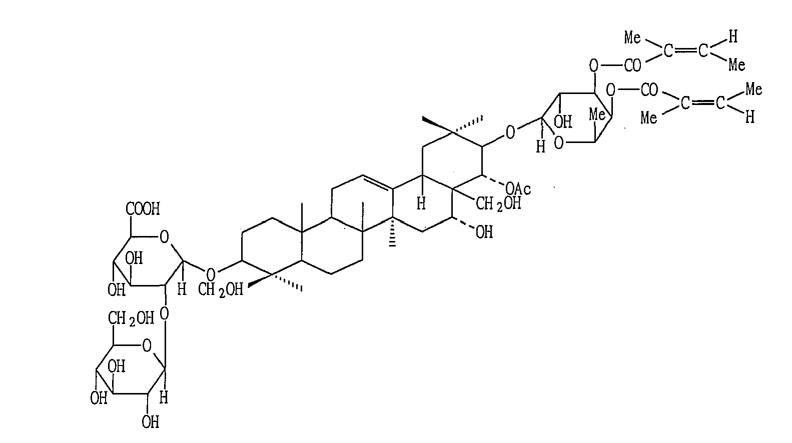

In Shinyleaf yellowhom and its extract, the ratio of saponin B (C₆₀H₉₂O₂₄) relative to total saponins is fairly constant. Saponin B accounts for 30 to 35% of the sum of saponins A, B, C and D. Thus, the inventor has discovered that the accurate determination of the content of saponin B in Shinyleaf yellowhom will enable the calculation of the relative content of total saponins.

The rationale of determining the content of total saponins is: 1g of Shinyleaf yellowhorn extract obtained by the extraction method of the present invention contains more than 150mg of the saponin B (C₆₀H₉₂O₂₄), i.e., the content of Shinyleaf yellowhorn saponin B exceeds 15%. Accordingly, given the fact that saponin B accounts for 30% of the sum of saponins A, B, C and D, 1g of Shinyleaf yellowhorn extract contains more than 500mg of total saponins, i.e., the content of total saponins in Shinyleaf yellowhorn extract exceeds 50%, calculated from the content of saponin B.

In the present invention, the total saponins accounts for over 50% of the total Shinyleaf yellowhorn extract, based on dry mass. Meanwhile, saponin B(C₆₀H₉₂O₂₄) accounts for 30~35% of the total saponins. Accordingly, the content of saponin B in the dried Shinyleaf yellowhorn extract is more than 15%. The method for preparing Shinyleaf yellowhorn extract disclosed in the present invention is stable and reliable, and can yield an extract containing high content of total saponins, thereby enable the stable quality of pharmaceutical preparations, the decreased drug dosage, the increased therapeutic effect; and the ease for pharmaceutical preparations.

By showing the effects of Shinyleaf yellowhorn extract on the urination frequency of the water-loaded m ice, on the excised smooth muscle of bladder of rats, and on the bioelectricity of the extracellular electrode of the external sphincter of urinary bladder of rabbits, as well as its antagonistic action on the Ach (acetylcholine) induced contraction of the excised smooth muscle of bladder of rats, the present invention has demonstrated that Shinyleaf yellowhorn extract is effective in the treatment of the urinary incontinence and frequent micturition due to senile dementia or urinary bladder degradation. By showing the effects of Shinyleaf yellowhorn extract on the sleeping time of mice, the present invention has proven that Shinyleaf yellowhom extract is effective in the treatment of children enuresis. By showing the improving effects of Shinyleaf yellowhom extract on the anisodine-induced rat memory impairment, and its effect on the avoidance conditioned reflex of the dementia rat model with destructed cholinergic neuron and Meynert basal ganglia, the present invention has demonstrated the efficacy of Shinyleaf yellowhom extract in the treatment of dementia. It has been proven that Shinyleaf yellowhorn extract disclosed in the present invention has the effects of improving the brain function and enhancing the brain activity, and can be used for treating the urinary incontinence and frequent micturition due to senile dementia or urinary bladder degradation, children enuresis, pediatric mental retardation, brain aging, middle-aged and senile dementia and Parkinson's syndrome.

The plant extract obtained by the method of the present invention can be used, together with the pharmaceutically acceptable carriers, for manufacturing drug compositions, for example in the form of tablet, capsule, oral liquid, injection, and drop pill. The conventional drug production methods in the pharmaceutical field can be used for the production of various pharmaceutical preparations with the present plant extract. The pharmaceutical preparation is preferably for oral or parenteral administration, more preferably for oral administration.

The drug composition disclosed in the present invention can be administrated orally as any type of pharmaceutical preparations suitable for oral administration, including but not limited to capsule, tablet, aqueous suspension and solution. In the case of the oral tablet, the commonly used carriers include lactose and corn starch, and usually added with lubricants such as magnesium stearate. As for orally administered capsules, the suitable diluents include lactose and dried corn starch. As for the orally administered aqueous suspension, the active ingredients are combined with the emulsifying and suspending agents. If necessary, sweeting agent, or flavoring agent or colorant can be added to the drug composition.

In a preferable embodiment of the present invention, the orally acceptable dosage form contains 10~90% (preferably 40~80%) of Shinyleaf yellowhom extract. If calculated in terms of Shinyleaf yellowhom total saponins, the content is 5~80% (preferably 20~40%). The dosage form is preferably administrated for 1∼4 (preferably 2~3) times per day, each dose of 0.1~1g (preferably 0.2~0.4g).

The sterile injectable forms of the drug composition disclosed in the present invention can be the aqueous or oily suspensions. These suspensions can be prepared with the suitable dispersing or wetting agents together with the suspending agent by established techniques in the art. The sterile injectable pharmaceutical preparations may also be the sterile injectable liquor or suspension in the nontoxic, parenterally acceptable diluent agent or solvent, such as a solution of 1,3-butanediol. The acceptable carriers and solvents include water, Ringer's solution and isotonic sodium chloride solution. In addition, the sterile fixed oil is often used as solvent or suspension medium.

If necessary, the maintenance dose of the present compound, composition or combinations thereof can be prescribed for the patient once their health status is improved. Subsequently, the dose and/or frequency of drug administration can be reduced to the minimum level required to maintain the improved health status. Once the improvement of the symptoms reaches the required level, the treatment should be discontinued. However, once the symptoms of the disease have any recurrence, the patient may need intermittent treatments over a long period.

The pharmaceutical preparations disclosed in the present invention may also contain, or may be used in combination with or in proper order with, other active agent or drugs for urinary incontinence and frequent micturition due to senile dementia or urinary bladder degradation, children enuresis, pediatric mental retardation, brain aging, middle-aged and senile dementia and Parkinson's syndrome.

It is also noted that the specific dose of drug administration and the specific treatment regime for a specific patient depend on a variety of factors, including the activity of the specific extract, the age, the body weight, the general health status, the gender, the diet of the patient, the time of administration, the excretion rate, the drug composition, the judgment of the attending physician and the severity of the specific disease to be treated.

### The preferred embodiments the present invention

The following examples are used for the further illustration of, but not the limitation of, the present invention.

### Example I: Effects of different solvents in the preparation of Shinyleaf yellowhorn extract cream

Raw materials of Shinyleaf yellowhom is dried and deoiled (preferably by squeezing with an oil press) to get Shinyleaf yellowhorn cake powder. 0.15g of the cake powder is cccurately weighted and extracted with a twice weight of Extractant A. The extraction is performed 4 times, each lasting 5 hours. The solvent is recovered under reduced pressure and dried to obtain Shinyleaf yellowhom extract.

Extractant A can be hexane, diethyl ether, chloroform, or petroleum ether. Extraction can also be conducted by supercritical CO₂ extraction procedure. The technical parameters for supercritical CO₂ extraction are as follows: extraction pressure: 25~30MPa; extraction temperature: 45°C; performed times: 4; entraining agent: ethanol, volume fraction: 70%, amount: 3%. Dry the resultant extract to obtain Shinyleaf yellowhorn extract cream.

By extracting Shinyleaf yellowhorn cake powder with the said solvents or extracted by the supercritical CO₂ extraction method, and measuring and comparing the respective results, we have found that all of those solvents can realize an extracting efficiency of 70% or more, while the supercritical CO₂ extraction method can realize 80% or more. Taking into account that the diethyl ether is an anesthetic and that the chloroform is highly toxic, and that the production of the petroleum ether is unsafe, it is more prefered to select N-hexane as Extractant A. Meanwhile, the supercritical CO₂ extraction method has a higher extraction yield, free of toxic impurities, and can be adopted instead of the conventional extraction method with Extractant A.

### Example II:

5kg of Shinyleaf yellowhorn cake powder is weighted and added with 10kg of petroleum ether for extraction. The extraction process is performed 4 times, each lasting 6 hours. The solvent is recovered under reduced pressure and the resultant liquid extract is dried to obtain Shinyleaf yellowhom extract cream. 1kg of the extract cream is weighted and added with 6L of the 55% methanol solution. The extraction process is performed 2 times, each lasting 2 hours. The liquid extracts is filtered and combined, and the solvent is recovered at 60°C under reduced pressure, until there is no odor of alcohols. The resultant extract is dissolved in 1L of water and then filtered to obtain Liquid Extract I. Liquid Extract I is further extracted with 1L of isobutanol for 3 times, each lasting 2 hours. The liquid extracts is filtered and combined, and then the solvent is recovered at 70°C under reduced pressure, until there is no odor of alcohols. The resultant extract is dissolved with 1L of water, then the solution is filtered to obtain Liquid Extract II. For the purpose of analysis, a portion of Liquid Extract II is evaporated to dryness to obtain a crude extract of Shinyleaf yellowhom.

### Example III:

5kg of Shinyleaf yellowhorn cake powder is weighted and added with 15kg of diethyl ether for extraction. The extraction process is performed 3 times, each lasting 5 hours. The solvent is recovered under reduced pressure and the resultant liquid extract is dried to obtain Shinyleaf yellowhom extract cream. 1kg of the extract cream is weighted and added with 7L of the 65% ethanol solution to perform extraction for 3 hours. The liquid extracts is filtered and combined, and the solvent is recovered at 65 °C under reduced pressure, until there is no odor of alcohols. The resultant extract is dissolved in 2L of water and then filtered to obtain Liquid Extract I. Liquid Extract I is further extracted with 2L of pentanol for 3 hours. The liquid extracts is filtered and combined, and then the solvent is recovered at 62 °C under reduced pressure, until there is no odor of alcohols. The resultant extract is dissolved with 0.5L of water, then the solution is filtered to obtain Liquid Extract II. For the purpose of analysis, a portion of Liquid Extract II is evaporated to dryness to obtain a crude extract of Shinyleaf yellowhorn.

### Example IV:

5kg of Shinyleaf yellowhorn cake powder is weighted and added with 15kg of petroleum ether for extraction. The extraction process is performed 5 times, each lasting 4 hours. The solvent is recovered under reduced pressure and the resultant liquid extract is dried to obtain Shinyleaf yellowhom extract cream. 1kg of the extract cream is weighted and added with 8L of the 75% ethanol solution for extraction. The extraction process is performed two times, each lasting 2 hours. The liquid extracts is filtered and combined, and the solvent is recovered at 70 °C under reduced pressure, until there is no odor of alcohols. The resultant extract is dissolved in 1L of water and then filtered to obtain Liquid Extract I. Liquid Extract I is further extracted with 2L of n-butanol for 2 times, each lasting 4 hours. The liquid extracts is filtered and combined, and then the solvent is recovered at 65 °C under reduced pressure, until there is no odor of alcohols. The resultant extract is dissolved with 0.7L of water, then filtered to obtain Liquid Extract II. For the purpose of analysis, a portion of Liquid Extract II is evaporated to dryness to obtain a crude extract of Shinyleaf yellowhom.

### Example V:

5kg of Shinyleaf yellowhom cake powder is weighted and added with 10kg of petroleum ether for extraction. The extraction process is performed 3 times, each lasting 6 hours. The solvent is recovered under reduced pressure and the resultant liquid extract is dried to obtain Shinyleaf yellowhom extract cream. 1kg of the extract cream is weighted and added with 4L of the 85% methanol solution to perform extraction for 4 hours. The liquid extracts is filtered and combined, and the solvent is recovered at 67°C under reduced pressure, until there is no odor of alcohols. The resultant extract is dissolved in 2L of water and then filtered to obtain Liquid Extract I. Liquid Extract I is further extracted with 1L of n-propanol for 3 times, each lasting 2~4 hours. The liquid extracts is filtered and combined, and then the solvent is recovered at 60°C under reduced pressure, until there is no odor of alcohols. The resultant extract is dissolved with 1L of water, then filtered to obtain Liquid Extract II. For the purpose of analysis, a portion of Liquid Extract II is evaporated to dryness to obtain a crude extract of Shinyleaf yellowhom.

### Example VI:

5kg of Shinyleaf yellowhom cake powder is weighted and added with 10kg of n-hexane for extraction. The extraction process is performed 4 times, each lasting 6 hours. The solvent is recovered under reduced pressure and the resultant liquid extract is dried to obtain Shinyleaf yellowhorn extract cream. 1kg of the extract cream is weighted and added with 6L of the 55% methanol solution. The Extraction performed 2 times, each lasting 2 hours. The liquid extracts is filtered and combined, and the solvent is recovered at 60 °C under reduced pressure, until there is no odor of alcohols. The resultant extract is dissolved in 1L of water and then filtered to obtain Liquid Extract I. Liquid Extract I is further extracted with 1L of isobutanol for 3 times, each lasting 2 hours. The liquid extracts is filtered and combined, and then the solvent is recovered at 70°C under reduced pressure, until there is no odor of alcohols. The resultant extract is dissolved with 1L of water, then filtered to obtain Liquid Extract II. For the purpose of analysis, a portion of Liquid Extract II is evaporated to dryness to obtain a crude extract of Shinyleaf yellowhorn.

### Example VII:

5kg of Shinyleaf yellowhorn cake powder is weighted and added with 15kg of n-hexane for extraction. The extraction process is performed 3 times, each lasting 5 hours. The solvent is recovered under reduced pressure and the resultant liquid extract is dried to obtain Shinyleaf yellowhom extract cream. 1kg of the extract cream is weighted and added with 7L of the 65% ethanol solution to perform extraction for 3 hours. The liquid extracts is filtered and combined, and the solvent is recovered at 65°C under reduced pressure, until there is no odor of alcohols. The resultant extract is dissolved in 2L of water and then filtered to obtain Liquid Extract I. Liquid Extract I is further extracted with 2L of pentanol for 3 hours. The liquid extracts is filtered and combined, and then the solvent is recovered at 62 °C under reduced pressure, until there is no odor of alcohols. The resultant extract is dissolved with 0.5L of water, then filtered to obtain Liquid Extract II. For the purpose of analysis, a portion of Liquid Extract II is evaporated to dryness to obtain a crude extract of Shinyleaf yellowhom.

### Example VIII:

5kg of Shinyleaf yellowhorn cake powder is weighted and added with 15kg of n-hexane for extraction. The extraction process is performed 5 times, each lasting 4 hours. The solvent is recovered under reduced pressure and the resultant liquid extract is dried to obtain Shinyleaf yellowhom extract cream. 1kg of the extract cream is weighted and added with 8L of the 75% ethanol solution. The extraction is performed 2 times, each lasting 2 hours. The liquid extracts is filtered and combined, and the solvent is recovered at 70 °C under reduced pressure, until there is no odor of alcohols. The resultant extract is dissolved in 1L of water and then filtered to obtain Liquid Extract I. Liquid Extract I is further extracted with 2L of n-butanol, the extraction performed 2 times, each lasting 4 hours. The liquid extracts is filtered and combined, and then the solvent is recovered at 65°C under reduced pressure, until there is no odor of alcohols. The resultant extract is dissolved with 0.7L of water, then filtered to obtain Liquid Extract II. For the purpose of analysis, a portion of Liquid Extract II is evaporated to dryness to obtain a crude extract of Shinyleaf yellowhom.

### Example IX:

5kg of Shinyleaf yellowhorn cake powder is weighted and added with 10kg of n-hexane for extraction. The extraction process is performed 3 times, each lasting 6 hours. The solvent is recovered under reduced pressure and the resultant liquid extract is dried to obtain Shinyleaf yellowhom extract cream. 1kg of the extract cream is weighted and added with 4L of the 85% methanol solution to perform extraction for 4 hours. The liquid extracts is filtered and combined, and the solvent is recovered at 67°C under reduced pressure, until there is no odor of alcohols. The resultant extract is dissolved in 2L of water and then filtered to obtain Liquid Extract I. Liquid Extract I is further extracted with 1 L of isopropanol, the extraction performed 3 times, each lasting 2~4 hours. The liquid extracts is filtered and combined, and then the solvent is recovered at 60 °C under reduced pressure, until there is no odor of alcohols. The resultant extract is dissolved with 1L of water, then filtered to obtain Liquid Extract II. For the purpose of analysis, a portion of Liquid Extract II is evaporated to dryness to obtain a crude extract of Shinyleaf yellowhom.

### Comparative Example I:

5kg of Shinyleaf yellowhom cake powder is weighted and added with 10kg of water for extraction. The extraction process is performed 4 times, each lasting 5 hours. The solvent is recovered under reduced pressure and the resultant liquid extract is dried to obtain Shinyleaf yellowhom extract cream. 1kg of the extract cream is weighted and added with 6L of the 70% methanol solution. The extraction process is performed 2 times, each lasting 2 hours. The liquid extracts is filtered and combined, and the solvent is recovered at 60~70°C under reduced pressure, until there is no odor of alcohols. The resultant extract is dissolved in 2L of water and then filtered to obtain Liquid Extract I. Liquid Extract I is further extracted with 1L of n-butanol, the extraction performed 3 times, each lasting **2~4** hours. The liquid extracts is filtered and combined, and then the solvent is recovered at 65°C under reduced pressure, until there is no odor of alcohols. The resultant extract is dissolved with 1L of water, then filtered to obtain Liquid Extract II. For the purpose of analysis, a portion of Liquid Extract II is evaporated to dryness to obtain a crude extract of Shinyleaf yellowhom.

### Comparative Example II:

5kg of Shinyleaf yellowhom cake powder is weighted and added with 10kg of methanol for extraction. The extraction process is performed 4 times, each lasting 5 hours. The solvent is recovered under reduced pressure and the resultant liquid extract is dried to obtain Shinyleaf yellowhom extract cream. 1kg of the extract cream is weighted and added with 6L of n-butanol solution. The extraction process is performed 2 times, each lasting 2 hours. The liquid extracts is filtered and combined, and the solvent is recovered at 60~70°C under reduced pressure, until there is no odor of alcohols. The resultant extract is dissolved in 2L of water and then filtered to obtain Liquid Extract I. Liquid Extract I is further extracted with 1L of water, the extraction is performed 3 times, each lasting 2~4 hours. The liquid extracts is filtered and combined, and then the solvent is recovered at 65 °C under reduced pressure. The resultant extract is dissolved with 1L of water, then filtered to obtain Liquid Extract II. For the purpose of analysis, a portion of Liquid Extract II is evaporated to dryness to obtain a crude extract of Shinyleaf yellowhorn.

### Refining Example I:

Liquid Extract II obtained from Example VI is loaded onto styrene macroporous resin column D201 and adsorbed for 20min. The column is washed with 1500ml of water at a flow rate of 3.0ml/min and then eluted with 1500ml of 35% methanol or ethanol solution as the eluting agent at a flow rate of 1.5ml/min. A 20% alcohol eluate is collected and the solvent is recovered under reduced pressure at 68°C until there is no odor of alcohols. The resultant extract is concentrated and dried to obtain the refined extract of Shinyleaf yellowhorn.

### Refining Example II:

Liquid Extract II obtained from Example VII is loaded onto styrene macroporous resin column AB8 and adsorbed for 30min. The column is washed with 1300ml of water at a flow rate of 4.0ml/min and then eluted with 1000ml of 80% methanol or ethanol solution as the eluting agent at a flow rate of 2ml/min. A 30% alcohol eluate is collected and the solvent is recovered under reduced pressure at 65 °C until there is no odor of alcohols. The resultant extract is concentrated and dried to obtain the refined extract of Shinyleaf yellowhorn.

### Refining Example III:

Liquid Extract II obtained from Example VIII is loaded onto styrene macroporous resin column HP20 and adsorbed for 35min. The column is washed with 900ml of water at a flow rate of 1.0ml/min and then eluted with 3000ml of 60% methanol or ethanol solution as the eluting agent at a flow rate of 1ml/min. A 25% alcohol eluate is collected and the solvent is recovered under reduced pressure at 60 °C until there is no odor of alcohols. The resultant extract is concentrated and dried to obtain the refined extract of Shinyleaf yellowhorn.

### Refining Example IV:

Liquid Extract II obtained from Example IX is loaded onto styrene macroporous resin column D101 and adsorbed for 40min. The column is washed with 600ml of water at a flow rate of 2.0ml/min and then eluted with 2000ml of 40% methanol or ethanol solution as the eluting agent at a flow rate of 0.5ml/min. A 22% alcohol eluate is collected and the solvent is recovered under reduced pressure at 60 °C until there is no odor of alcohols. The resultant extract is concentrated and dried to obtain the refined extract of Shinyleaf yellowhorn.

### Analyzing Example: Determination of Content of Shinyleaf yellowhorn Saponin B

Determine the content of saponin B by HPLC.

Preparation of control sample: Shinyleaf yellowhom extract cream is extracted with 55~85% methanol or ethanol, and the resultant liquid extract is then loaded onto the styrene macroporous resin column for absorption. Eluted with 20~80% ethanol solution as eluting agent. A 50~70% alcohol eluate is collected and loaded onto the silica column. Eluted with chloroform/methanol as the eluting agent. A 45:55 (V/V, chloroform/methanol) of the eluate is collected and chromatographed with a preparative liquid chromatograph, using methanol/water (V/V of 75/25) as the mobile phase. The resultant eluate concentrated and dried to obtain the control sample of saponin B. As determined by the evaporative light detector for liquid chromatography, the purity of the saponin B control sample is more than 97%.

Chromatographic detecting conditions and system adaptability tests: ODS used as the loading agent, and the methanol-water-phosphoric acid (with V/V/V of 75/25/0.01) used as the mobile phase; the detection wavelength is 215 nm; and the number of theoretical plates (N.T.P) shall be no less than 3000 calculated according to the peak of Shinyleaf yellowhorn saponin B.

Preparation of the control sample solution: Some saponin B control sample is accurately weighed and mixed in the mobile phase to prepare the 0.04mg/ml solution.

Preparation of the test sample solution: 4g of Shinyleaf yellowhom extract is weighted and ultrasonic extracted with 70% ethanol; A 10ml portion is evaporated to dryness; then dissolved in water and loaded onto the resin column; according to 'column chromatography' test, a 70% ethanol eluate is collected and then filtered with a 0.45µm filter membrane to obtain the test sample solution.

Determination method: a 10µl of the control sample solution and the test sample solution each is drawn and determined the peak area for the calculation of content of saponin B.

### Comparison of saponin B contents in the extracts prepared by various extraction methods

The content of saponin B in Shinyleaf yellowhom extract obtained from each example is determined with the above method, and then the contents of total saponins are extrapolated assuming that saponin B accounts for 30% of total saponins. The results are as follows.

**Table 1. Contents of saponin B and total saponins in Shinyleaf yellowhom extracts**

| Example No. from which the crude extract of Shinyleaf yellowhom obtained | saponin B | total saponins |
|---|---|---|
| Example II | 6.03% | 20.1% |
| Example III | 5.55% | 18.5% |
| Example IV | 7.44% | 24.8% |
| Example V | 6.75% | 22.5% |
| Example VI | 6.18% | 20.6% |
| Example VII | 5.73% | 19.1% |
| Example VIII | 7.59% | 25.3% |
| Example IX | 6.87% | 22.9% |
| Comparative Example I | 1.38% | 4.6% |
| Comparative Example II | 2.16% | 7.2% |
| Refining Example I | 24.69% | 82.3% |
| Refining Example II | 27.54% | 91.8% |
| Refining Example III | 22.05% | 73.5% |
| Refining Example IV | 16.68% | 55.6% |

### Experimental Example I: effect of Shinyleaf yellowhorn extract on urination frequency of water-load mice

The experimental animal strains used herein are the SPF KM mice and Wistar rats. Each rat or mouse receives gavage drug administration. Shinyleaf yellowhorn extract is the refined extract from Refining Example IV, in the form of capsules. The control drug is imipramine, which has a strong anti-depression action and a weak atropine-like action, and is often used for treatment of the depression and children enuresis. The experimental method is calculating the urination frequency of mice based on the colorable reaction taking place when the dried potassium iodide and starch are mixed with the urine.

**Table 2. Effects of Shinyleaf yellowhorn Extract on Urination frequency of Water-load Mice**

| Group | Number of animal | Dose | Urination frequency (times/each) X±SD | | | |
|---|---|---|---|---|---|---|
| | (n) | (g/kg*d) | 0.5h | 1.0h | 2.0h | 3.0h |
| physiologic saline | 10 | Equi. volume×10 | 1.2±1.1 | 2.9±1.4 | 2.4±1.6 | 1.2±1.1 |
| Imipra High-dose extract mine | 10 | 0.01×10 | 0.9±0.7 | 2.9±1.3 | 2.6±1.5 | 1.3±0.8 |
| Low-dose extract | 10 | 0.01×10 | 1.0±0.7 | 2.4±1.3 | 2.0±1.5 | 1.4±1.1 |
| High-dose extract | 10 | 0.20×10 | 0.8±0.6 | 1.9±1.0* | 1.9±1.6 | 0.8±0.7 |

| | | | | | | |
|---|---|---|---|---|---|---|
| *: P<0.05, compared with control group | | | | | | |

The experimental results show that the administration of a certain dose of Shinyleaf yellowhom extract will lead to the decrease in the urination frequency of the water-load mice within a certain period of time.

### Experimental Example II: Effects of Shinyleaf yellowhorn extract and imipramine on excised smooth muscle of bladder of rats

The experimental animal strain used herein is the SPF Wistar rats. The experiment process is as follows: the rat bladder is surgically excised to prepare the strips of smooth muscle; place the smooth muscle in the thermostat nutrient solution, and then added with Shinyleaf yellowhorn extract (the refined extract from Refining Example IV, in the form of capsules) and the imipramine respectively, under the oxygen supply conditions, to observe the changes in smooth muscle.

Criteria for grading: after the calibration by a transducer, a 0.5mm fall of the baseline indicates the complete relaxation of the smooth muscle, an unchanged baseline indicates the non-response of the smooth muscle, while a rise of the baseline shows the contraction of the smooth muscle.

**Table 3. Concentrations of solutions as smooth muscle shows complete relaxation**

| Group | Number of times of experiment | Concentration of solution (mg/ml) |
|---|---|---|
| Imipramine | 12 | 0.15 |
| Shinyleaf yellowhom extract | 12 | 0.80 |

The experimental results show that Shinyleaf yellowhom extract can directly act on the smooth muscle of bladder of rats, making it relax.

### Experimental Example III: the antagonistic action of Shinyleaf yellowhorn extract on Ach induced contraction of the excised smooth muscle of the bladder of rats

The experimental method is same as that described in Experimental Example II.

**Table 4. EC50 Determination of the action of Shinyleaf yellowhom extract and/or Ach on the contraction of the excised bladder smooth muscle of the rat**

| Drug | EC₅₀ (mg/ml) | P (f=∞) (t=2.5) |
|---|---|---|
| Ach | 3.4×10⁻¹¹ | --- |
| Shinyleaf yellowhom extract plus Ach | 2.9×10⁻¹⁰ | <0.05 |

The experimental results show that Shinyleaf yellowhom extract (the refined extract from Refining Example IV, made into the form of capsules) has the antagonistic action on the Ach induced contraction of the excised bladder smooth muscle of the rat, making the contracted smooth muscle completely relaxed.

### Experimental Example IV: Effects of Shinyleaf yellowhorn extract on the bioelectricity of the extracellular electrode of the external bladder sphincter of the rabbit

Experimental method: surgically implant indwelling electrode and record the changes of the external bladder sphincter of the rabbit with a physiological recorder. Administration mode: gavage administration. The adrenomimetic drug Midodrine is a selective α₁ adrenoceptor agonist, commonly used for treating of stress incontinence clinically.

**Table 5. Effects of Shinyleaf yellowhom extract on the external bladder sphincter of the rabbit (X±SD)**

| group | animals (n) | Dose | Frequency (times/min) | Wave amplitude (mv) | Time duration (min) |
|---|---|---|---|---|---|
| Control group | 30 | | 5.5±3.0 | 4.2±1.8 | 0.2±0.1 |
| Midodrine | 10 | 0.4mg/kg | 35.4±11.6** | 14.9±6.7** | 18.7±10.3 ** |
| Low-dose | 10 | 0.1 g/kg | 25.6±10.1** | 9.7±4.2** | 8.2±3.6** |
| High-dose | 10 | 0.2g/kg | 31.3±12.8** | 13.9±5.4** | 12.6±6.3** |

| | | | | | |
|---|---|---|---|---|---|
| *: P<0.05, Compared with the control group. **: P<0.01, Compared with the control group. | | | | | |

The experimental results show that both of Shinyleaf yellowhorn extract (the refined extract from Refining Example IV, in the form of capsules) and the Midodrine can significantly raise the frequency and wave amplitude of the bioelectricity of the extracellular electrode of the external bladder sphincter of the rabbit, thus proving their effect in significantly enhancing the excitability of the bladder sphincter.

### Experimental Example V: Effect of Shinyleaf yellowhorn extract on the sleeping hours of the mouse

Establish the animal model of sleeping of the mouse by an intraperitoneal injection of sodium pentobarbital. Administer drug by gavage administration one hour prior to the injection, so as to observe the recovery time for the righting reflex of the mouse. Administration mode: gavage administration. The telucidone (meclofenoxate, or centrofenoxine) has an exciting action on central nerve and commonly used for the clinical treatment of traumatic unconscious disturbance and children enuresis.

**Table 6. Effect of Shinyleaf yellowhom extract on the sleep hours of mice (X±SD)**

| group | animals (n) | Dose (g/kg) | Hour of sleep (min) |
|---|---|---|---|
| sleeping control | 15 | | 78.6±15.3 |
| Telucidone | 15 | 0.02 | 61.2±10.7* |
| Low-dose extract | 15 | 0.10 | 63.6±11.2* |
| High-dose extract | 15 | 0.20 | 57.5±9.4** |

| | | | |
|---|---|---|---|
| *: P<0.05, compared with sleeping control group **: P<0.01, compared with sleeping control group | | | |

The experimental results show that Shinyleaf yellowhom extract (the refined extract from Refining Example IV, in the form of capsules) can significantly shorten the sodium pentobarbital prolonged sleep hours in mouse, indicating that it can significantly enhance the excitability of central nerve.

### Experimental Example VI: Effects of Shinyleaf yellowhorn extract on memory impairment

Establish the animal model of memory impairment pursuant to the method disclosed in "Memory Impairment Animal Models" (Pharmacological Experimental Methodology, Third Edition, P829). Anisodine is an anti-cholinergic drug, toxicity smaller than that of atropine.

Administration mode: gavage administration. The cholinomimetic drug Donepezil (Aricept) is a cerebral metabolism nootropics and a specific reversible inhibitor of acetylcholinesterase. The drug can slow down the decomposition of acetylcholine in the synaptic cleft, thus enhancing the content of acetylcholine, and commonly used for clinical treatment of the Alzheimer's senile dementia.

**Table 7. Improving effect of Shinyleaf yellowhom extract on anisodine-induced memory impairment of mice (X±SD)**

| group | animals (n) | Dose | Electricity-avoiding latency (s) | Number of error (times) |
|---|---|---|---|---|
| Normal control | 15 | | 260.3±133.9* | 0.18±0.16** |
| Model control | 15 | 5mg/kg | 88.7±62.4 | 1.37±1.10 |
| Aricept | 15 | 0.5mg/kg | 255.9±126.1** | 0.20±0.18** |
| Low-dose extract | 15 | 0.1g/kg | 181.4±103.6* | 0.48±0.35* |
| High-dose extract | 15 | 0.2g/kg | 224.3±118.0** | 0.28±0.21** |

| | | | | |
|---|---|---|---|---|
| *: P<0.05, compared with model control group ; **P<0.01, , compared with model control group | | | | |

The experimental results show that Shinyleaf yellowhom extract (the refined extract from Refining Example IV, in the form of capsules) can significantly prolong the electricity-avoiding time in mouse with memory impairment, and reduce the number of error, indicating that Shinyleaf yellowhom extract can significantly improve the acquired memory impairment.

### Experimental Example VII: Effect of Shinyleaf yellowhorn extract on avoidance conditioned reflex of the dementia rat model with destructed cholinergic neuron and Meynert basal ganglia.

The animal model of senile dementia is established pursuant to the method disclosed in "Experimental Alzheimer's Disease Animal Model with Destructed Cholinergic Neuron" (Pharmacological Experimental Methodology, Third Edition, P848). The kainic acid is a receptor agonist for the central exciting neurotransmitter glutamate, which has been widely used as a tool drug to establish the neurotoxicity model by the international community.

Administration mode: gavage administration. The Piracetam-NEGPF [piracetam, topiramate acetamide, amide pyrrole ring (ketone)] is a brain metabolism nootropics that can protect and repair cranial nerve cells, promote the transformation of ADP into ATP in the brain and the synthesis of acetylcholine and prevent against the brain function injury caused by the physical and chemical factors, and is used in clinic for the treatment of the brain function disorder due to aging, cerebral vascular accident, traumatic brain injury, and CO poisoning.

**Table 8 Effect of Shinyleaf yellowhorn extract on avoidance conditioned reflex of the dementia rat model X±SD**

| group | animals (n) | Dose (g/kg) | The first day | | The third day | | The fifth day | | 72h post training of stopping | |
|---|---|---|---|---|---|---|---|---|---|---|
| | | | Positive rate (%) | Latency (s) | Positive rate (%) | Latency (s) | Positive rate (%) | Latency (s) | Positive rate (%) | Latency (s) |
| Normal control | 10 | | 55.7±16.3* | 3.1±0.8** | 91.2±8.8** | 2.±0.6** | 100** | 1.3±0.4** | 92.3±7.4** | 1.7±0.5** |
| Dementia rat model | 8 | | 28.3±10.2 | 8.3±3.6 | 31.0±7.2 | 6.3±2.9 | 48.7±10.7 | 4.8±1.0 | 37.5±8.0 | 6.9±1.2 |
| Piracetam-NEGPF Shinyleaf | 7 | 0.2 | 30.5±12.7 | 7.1±3.3 | 57.6±8.0* | 4.4±1.0* | 71.05±8.2* | 2.6±0.6* | 58.2±7.4* | 4.6±0.7* |
| yellowhorn extract | 7 | 0.2 | 28.8±11.4 | 7.8±2.6 | 52.1±8.5* | 5.2±1.4* | 68.4±9.6* | 3.1±1.1* | 51.7±6.6* | 5.1±0.9* |

| | | | | | | | | | | |
|---|---|---|---|---|---|---|---|---|---|---|
| *: P<0.05, compared with dementia rat model group; ** P<0.01, compared with dementia rat model group. | | | | | | | | | | |

The experimental results show that Shinyleaf yellowhom extract (the refined extract from Refining Example IV, in the form of capsules) can significantly enhance the positive rate of avoidance response in the dementia animal model and shorten the latency of avoidance response, which indicates that it can improve the brain function in dementia animal model.

### Experimental Example VIII: Clinical trial report of treatment of senile dementia with Shinyleaf yellowhorn extract

Case sources: A total of 30 outpatients, diagnosed with senile dementia, were selected randomly for analysis. The 30 subjects included 23 males (76.66%) and seven females (23.33%). Among them were 6 cases aged from 68 to 70 years old, 17 cases aged from 70 to 80 and seven aged from 80 to 86, with an average age of 77.5.

The diagnostic criteria for senile dementia, issued by the China Medical Association in 1989, were used for the diagnosis. Shinyleaf yellowhorn extract is obtained form Refining Example III and in the form of tablets. The dose is 0.2 grams per time (three times a day). After the successive administration for three months, the therapeutic effect is evaluated.

Criteria of therapeutic effect: the criteria of therapeutic effect as revised in the National Conference on Senile Dementia held by the Gerontological Society and the Internal Medical Society of the National Association of Traditional Chinese Medicines on May 1990 were used for the judgment of the therapeutic effect of the treatment:
Cure: The subjective symptoms have completely disappeared; the consciousness is clear; the orientation is sound; the subject can answer questions correctly and the response is sensitive; the subject has the ability of self-care, and is able to participate in social activities;
Marked efficacy: The main symptoms has disappeared; the consciousness is clear; the orientation is sound; the subject can answer questions substantially correctly and the response is fairly sensitive; the subject has the ability of self-care, and is able to participate in normal social activities;
Efficacy: The main psychiatric symptoms has diminished or partially disappeared; the subject has the ability of self-care; the subject can answer questions substantially correctly but the response is retarded; there are still some intellectual and personality disorders;
Inefficacy: The main psychiatric symptoms show no signs of improving, or the pathogenetic condition is still worsening; the subject is incapable of self-care, can not answer questions correctly, and the consciousness is completed retarded.
Analysis of efficacy results: After the successive administration for three months, the therapeutic effect is evaluated. Of the total of 30 subjects, nine (30%) showed marked efficacy, and 19 (63%) showed some efficacy, while two (7%) showed no sign of efficacy; the total efficacy rate is 93%.

### Experimental Example IX: Clinical trial of treatment of pediatric mental retardation with Shinyleaf yellowhorn extract

Case sources: A total of 30 outpatients, diagnosed to be pediatric mental retardation, were selected randomly for analysis. The 30 subjects included 18 males (60%) and 12 females (40%). Among them were 16 cases aged 5~8, and 14 aged 8~13, with an average age of 8.1. The grading of patient's condition is as follows: 3 with severe conditions, 14 medium and 13 mild, respectively.

**Table 9 Distribution of IQ in different age groups before treatment**

| | IQ | | |
|---|---|---|---|
| Age span | 20-34 | 35-49 | 50-70 |
| 5~8 | 2 | 7 | 7 |
| 8∼13 | 1 | 7 | 6 |

The diagnostic standard: according to China-Wechsler Young Chilren Scale of Intelligence(CWYCSI), if the IQ score is less than 70, the subject was diagnosed to have the mental retardation, which is further divided into three grades: severe (with an IQ sore of 20-34), medium (35~49) and light (50~70).

Shinyleaf yellowhorn extract is obtained from Refining Example III and in the form of tables. The dose is 0.1 grams each time (three times a day). After the successive administration for three months, the IQ is determined.

Analysis of efficacy results: After the successive administration for three months, the IQ is determined. 11 of the total of 30 subjects showed marked effect, accounting for 36.66%, and 2 (6.67%) showed effect, while 17 shows no effect, accounting for 56.66%. The total effective rate is 43.33%.

**Table 10 Distribution of IQ in different age groups after treatment**

| | IQ | | | |
|---|---|---|---|---|
| Age span | 20-34 | 35-49 | 50-70 | Over 70 |
| 5~8 | 1 | 4 | 8 | 3 |
| 8~13 | 1 | 5 | 7 | 1 |

All of the subjects showed no adverse reaction during treatment. Therefore, it is apparent that Shinyleaf yellowhorn extract disclosed in the present invention has therapeutic effect in treating pediatric mental retardation, and is safe and reliable.

## Claims

1. Shinyleaf yellowhorn extract, comprising mainly Shinyleaf yellowhom total saponins, which accounts for more than 50%, preferably more than 60%, more preferably more than 70%, and most preferably more than 8 0% of the total weight of the dried Shinyleaf yellowhorn extract.

2. The Shinyleaf yellowhom extract according to Claim 1, wherein saponin B accounts for more than 15%, preferably more than 20%, and more preferably more than 25% of the total weight of the dried Shinyleaf yellowhom extract.

3. Method for preparing Shinyleaf yellowhom extract, comprising the following steps:
A. Raw materials of Shinyleaf yellowhornis dried and deoiled (preferably by squeezing with an oil press) to prepare the cake powder of Shinyleaf yellowhom, the cake powder is extracted with Extractant A or using supercritical CO₂ extraction method, the resultant extract is dried to obtain Shinyleaf yellowhom extract cream, wherein, extractant A is selected from n-hexane, petroleum ether, chloroform and diethyl ether, with n-hexane prefered;
B. Shinyleaf yellowhorn extract cream is further extracted with Extractant B, with the resultant extract filtered and combined, and then dissolved in water and filtered again to obtain Liquid Extract I, wherein extractant B is methanol or ethanol;
C. Liquid Extract I is further extracted with Extractant C, with the resultant extract filtered and combined, and then dissolved in the water and filtered again to obtain Liquid Extract II, wherein extractant C is selected from n-butanol, n-propanol, isopropanol, isobutanol, and pentanol, with n-butanol, n-propanol, or isopropanol prefered;
D. Liquid Extract II is loaded onto a styrene macroporous resin column for aborption, Then eluted by using the methanol or ethanol solution as the eluent, the eluate is collected, concentrated and dried to obtain the refined extract of Shinyleaf yellowhom.

4. The method according to Claim 3, in Step A, when extracted with Extractant A, it is prefered to add Extractant A to the cake powder at a weight/weight ratio of 1~5:1 (preferably 2~3:1), and perform the extraction process 2~6 times, preferably 3~5 times, then the solvent is recovered under reduced pressure and the resultant extract is dried to obtain Shinyleaf yellowhom extract cream.

5. The method according to Claim 3, in Step A, when extracted by the supercritical CO₂ extraction method, the cake powder is put in the supercritical CO₂ for the extraction, the technical parameters for the supercritical CO₂ extraction are as follows: the extraction pressure is 10~50 MPa (preferably 20~30MPa), the extraction temperature is 20~60°C (preferably 40~50°C), the number of extraction times is 2~5, the entraining agent is ethanol with a volume fraction of 60~95% and the amount of entraining agent used is 1~6% (preferably 2~4%); the resultant extract is dried to obtain Shinyleaf yellowhorn extract cream.

6. The method according to Claim 3, in Step B, when extracting Shinyleaf yellowhom extract powder with Extractant B, it is prefered to use Extractant B with a concentration of 50~90%, preferably 55~85%, or more preferably 65~80% at a volume/weight ratio of 2~9:1 (preferably 4~8:1), the extraction process is performed 1∼2 times, each lasting 2~4 hours, the resultant liquid extracts is filtered and combined, and then the solvent is recovered under reduced pressure preferably at 60~70°C, until there is no odor of alcohols; the resultant extract is dissolved in 0.5~3 volumes, preferably 1∼2 volumes of water and then filtered to obtain Liquid Extract I.

7. The method according to Claim 3, in Step C, for the further extraction of Liquid Extract I with Extractant C, it is prefered to use 0.5~3 volumes, preferably 1~2 volumes of Extractant C, and extract 1~3 times, each for 2~4 hours; then the resultant extract is filtered and combined, and the solvent is recovered under reduced pressure, preferably at 60~70°C, until there is no odor of alcohols; the resultant extract is dissolved in 0.2~2 volumes, preferably 0.5~1 volume of water, and filtered to obtain Liquid Extract II.

8. The method according to Claim 3, in Step D, when Liquid Extract II is loaded onto the styrene macroporous resin column for absorption, it is prefered to perform absorption for 10~60 minutes (preferably 20∼40 minutes), and thenthe column is washed with 600~1500ml of water at an prefered flow rate of 1.0~4.0ml/min, then eluted by using 800~3000ml of the methanol or ethanol solution with a concentration of 30~80%, preferably 40~70%, or more preferably 60~70% as eluent, at a prefered flow rate of 0.5 to 2.0ml/min; the 20~30% alcohol eluate is collected, and the solvent is recovered under reduced pressure, preferably at 60~70°C, until there is no odor of alcohols; the resultant solution is concentrated and dried to obtain the refined extract of Shinyleaf yellowhom.

9. The method according to Claim 8, in Step D, the styrene macroporous resin column to be used can be polar, or weak polar, or nonpolar.

10. The method according to any of Claims 3~8, wherein the extraction method is thermal reflux extraction or ultrasonic extraction.

11. Shinyleaf yellowhorn extract prepared by the method according to any of Claims 3~8, consists mainly of Shinyleaf yellowhom total saponins, which accounts for more than 50%, preferably more than 60%, more preferably more than 70%, and most preferably more than 80% of the total weight of the dried Shinyleaf yellowhorn extract.

12. A drug composition comprising Shinyleaf yellowhom extract and pharmaceutically acceptable carriers for treatment of the urinary incontinence and frequent micturition due to senile dementia or urinary bladder degradation, children enuresis, pediatric mental retardation, brain aging, middle-aged and senile dementia and Parkinson's syndrome, wherein Shinyleaf yellowhom extract consists mainly of Shinyleaf yellowhom total saponins, which accounts for more than 50%, preferably more than 60%, more preferably more than 70%, and most preferably more than 80% of the total weight of the dried Shinyleaf yellowhom extract.

13. The drug composition according to Claim 12, wherein Shinyleaf yellowhom extract is prepared by the methods according to any of Claims 3~8.

14. The use of Shinyleaf yellowhorn extract in preparation of drugs for the treatment of the urinary incontinence and frequent micturition due to senile dementia or urinary bladder degradation, children enuresis, pediatric mental retardation, brain aging, middle-aged and senile dementia and Parkinson's syndrome, the said Shinyleaf yellowhom extract mainly consists of Shinyleaf yellowhom total saponins, which accounts for more than 50%, preferably more than 60%, more preferably more than 70%, and most preferably more than 80% of the total weight of the dried Shinyleaf yellowhorn extract.

15. The use according to Claim 14, wherein Shinyleaf yellowhorn extract is prepared by the method according to any of Claims 3~8.
